Europäisches Patentamt

European Patent Office

Office européen des brevets

(19)

(11) EP 0 466 565 B1

(12) FASCICULE DE BREVET EUROPEEN

(45) Date de publication et mention
de la délivrance du brevet:
**22.05.1996 Bulletin 1996/21**

(51) Int Cl.6: **C07K 2/00**, G01N 33/74,
G01N 33/543, G01N 33/577

(21) Numéro de dépôt: **91401876.7**

(22) Date de dépôt: **05.07.1991**

(54) **Trousse pour le dosage spécifique de l'angiotensine II**

Satz zur spezifischen Bestimmung von Angiotensin-II

Kit for the specific determination of Angiotensin II

(84) Etats contractants désignés:
**AT BE CH DE DK ES FR GB GR IT LI LU NL SE**

(30) Priorité: **05.07.1990 FR 9008563**

(43) Date de publication de la demande:
**15.01.1992 Bulletin 1992/03**

(73) Titulaire: **SANOFI**
**F-75008 Paris (FR)**

(72) Inventeurs:
• **Simon, Dominique**
**F-34090 Montpellier (FR)**
• **Marchand, Jean**
**F-34080 Montpellier (FR)**
• **Badouaille, Gabriel**
**F-34570 Pignan (FR)**
• **Romestand, Bernard**
**F-34980 Saint-Gely-Du-Fesc (FR)**
• **Fehrentz, Jean-Alain**
**F-34400 Saint Nazaire de Pezan (FR)**

(74) Mandataire: **Gillard, Marie-Louise et al**
**Cabinet Beau de Loménie**
**158, rue de l'Université**
**F-75340 Paris Cédex 07 (FR)**

(56) Documents cités:
**EP-A- 0 273 453**

• **HORMONE AND METABOLIC RESEARCH, vol.
16, no. 11, novembre 1984, pages
606-610,Stuttgart, DE; J. NUSSBERGER et al.: A
simplified radioimmunoassay
forphysiologically active angiotensin peptides
[(1-8) octa- and '2-8)heptapeptides]"**

ANNEXE : SEQUENCE LISTING

(1) GENERAL INFORMATION :

    (i) APPLICANT:SANOFI

    (ii) TITLE OF INVENTION; Kit for the specific assay of Angiotensin II

    (iii) NUMBER OF SEQUENCES: 3

(2) INFORMATION FOR SEQ ID NO:1:

    (i) SEQUENCE CHARACTERISTICS:

        (A) LENGTH: 10 amino acids
        (B) TYPE: amino acid
        (D) TOPOLOGY: linear

    (ii) MOLECULE TYPE: peptide

    (vii) IMMEDIATE SOURCE:

        (B) CLONE: Angiotensin I

    (xi) SEQUENCE DESCRIPTION: SEQ ID NO:1:

```
        Asp Arg Val Tyr Ile His Pro Phe His Leu
        1               5                   10
```

(2) INFORMATION FOR SEQ ID NO:2:

    (i) SEQUENCE CHARACTERISTICS:

(A) LENGTH: 8amino acids
(B) TYPE: amino acid
(D) TOPOLOGY: linear

(ii) MOLECULE TYPE: peptide

(vii) IMMEDIATE SOURCE:

(B) CLONE: anti Ang III antibody immunogen

(ix) FEATURE:

(A) NAME/KEY: Modified-site
(B) LOCATION: 6..7
(D) OTHER INFORMATION: /label= Xaa
/note= "A direct bond or chain-link comprising 1 or 2 natural amino acids"

(ix) FEATURE:

(A) NAME/KEY: Modified-site
(B) LOCATION: 7..8
(D) OTHER INFORMATION: /label= Xaa /note= "A group of formula -NH-(CH(sub)2)(sub)n-CO-, in which n varies between 1 and 9"

(xi) SEQUENCE DESCRIPTION: SEQ ID NO:2:

```
Arg Val Tyr Ile His Xaa Xaa Cys
1               5
```

(2) INFORMATION FOR SEQ ID NO:3:

(i) SEQUENCE CHARACTERISTICS:

(A) LENGTH: 12 amino acids
(B) TYPE: amino acid
(D) TOPOLOGY: linear

(ii) MOLECULE TYPE: peptide

(iii) HYPOTHETICAL: NO

(vii) IMMEDIATE SOURCE:

(B) CLONE: anti Ang I antibody immunogen

(ix) FEATURE:

(A) NAME/KEY: Modified-site
(B) LOCATION: 2..3
(D) OTHER INFORMATION: /label= Xaa
/note= "A group of formula -NH-(CH(sub)2)(sub)n-CO, in which n varies between 1 and 9"

(ix) FEATURE:

(A) NAME/KEY: Modified-site
(B) LOCATION: 3..4
(D) OTHER INFORMATION: /label= Xaa
/note= "A direct bond or a chain-link comprising 1 or 2 natural amino acids"

(xi) SEQUENCE DESCRIPTION: SEQ ID NO:3:

```
Cys Xaa Xaa Arg Val Tyr Ile His Pro Phe His Leu
1                   5                   10
```

**Description**

La présente invention a pour objet une trousse pour le dosage immunologique spécifique de l'angiotensine II et le procédé pour l'immunodosage de l'angiotensine II utilisant ladite trousse.

L'angiotensine II est un puissant agent vasopresseur qui est le produit biologiquement actif du système rénineangiotensine : la rénine agit sur l'angiotensinogène du plasma pour produire un décapeptide (1-10), l'angiotensine I, celle-ci est convertie en un octapeptide (1-8), l'angiotensine II, par action de l'enzyme de conversion, puis une aminopeptidase produit un heptapeptide (2-8) : l'angiotensine III.

Les formules de ces différents peptides chez les mammifères sont données ci-après :

Angiotensine I, ci-dessous dénommée Ang I :

$$\overset{1}{\text{H-Asp-Arg-Val-Tyr-Ile-His-Pro-Phe-His-Leu-OH}}^{10} \qquad (I)$$

Angiotensine II, ci-dessous dénommée Ang II :

$$\overset{1}{\text{H-Asp-Arg-Val-Tyr-Ile-His-Pro-Phe-OH}}^{8} \qquad (II)$$

Angiotensine III, ci-dessous dénommée Ang III :

$$\overset{2}{\text{H-Arg-Val-Tyr-Ile-His-Pro-Phe-OH}}^{8} \qquad (III)$$

Une publication récente a montré que l'Ang III elle-même est dégradée en deux peptides (D.J. CAMBELL et al., J. Hypertens., 1990, 8, 165-172). Parmi ces peptides de dégradation, on peut citer en particulier un hexapeptide de formule :

$$\overset{3}{\text{H-Val-Tyr-Ile-His-Pro-Phe-OH}}^{8} \qquad (IV)$$

ci-dessous dénommé hexapeptide (IV) et un pentapeptide de formule :

$$\overset{4}{\text{H-Tyr-Ile-His-Pro-Phe-OH}}^{8} \qquad (V)$$

ci-dessous dénommé pentapeptide (V).

Ces peptides de dégradation sont présents dans le plasma humain. Il est vraisemblable qu'ils apparaissent également dans le plasma d'autres mammifères.

L'exploration hormonologique du système rénine-angiotensine est très importante pour le diagnostic et le traitement de l'hypertension artérielle. Actuellement, cette exploration effectuée en clinique se limite à la mesure de l'activité enzymatique de la rénine plasmatique, de l'activité de l'enzyme de conversion et à la mesure de la concentration plasmatique de la rénine active.

Le dosage direct de l'Ang II, qui est le peptide actif du système, est difficile à réaliser pour plusieurs raisons :

- très faible concentration plasmatique de l'Ang II (3 à 20 pg/ml chez le sujet normal, soit $3.10^{-12}$ à $2.10^{-11}$M) qui nécessite l'utilisation d'un anticorps Anti-Ang II très affin ;
- analogie de structure de l'Ang II avec différents peptides issus du clivage de l'angiotensinogène et présents dans le plasma, en particulier l'Ang I et surtout l'Ang III ou d'autres peptides de dégradation qui peuvent interférer dans le dosage.

Ainsi, J. NUSSBERGER et al. décrivent un dosage radio-immunologique qui ne permet pas de distinguer l'Ang II et III dans le plasma humain (Hormon. Metab. Res., 1984, 16 (II), 606-610 et J. Hypertens., suppl., 1988, 6 (4), S424-S425). Par ailleurs, la demande de brevet européen 273 453 décrit plusieurs anticorps monoclonaux anti-Ang II qui présentent tous une réactivité croisée avec l'Ang III ; la plus faible réactivité croisée, observée pour l'un d'entre eux, est de 32 %. Ainsi la réaction de l'Ang II avec cet anticorps n'est pas spécifique.

De plus, cet anticorps monoclonal a une affinité faible, de l'ordre de $10^9$M$^{-1}$, qui est insuffisante pour permettre un dosage dans le plasma (Biochem. Biophys. Res. Commun. 1987, 143, p. 133-139).

L'anticorps anti-Ang II, appelé 4D8 et décrit dans les actes du Congrès "The renin Angiotensin System as a Therapeutic Target" - Bâle, 29-31 octobre 1989, communication SANOFI, possède une forte affinité de l'ordre de $10^{11}M^{-1}$ ; mais il présente une réactivité croisée de l'ordre de 100 % avec l'Ang III et avec l'hexapeptide (IV) ; il présente une réactivité croisée avec le pentapeptide (V) d'environ 50 %. En fait, il est très difficile, et non connu à ce jour, d'avoir un anticorps à la fois très affin pour l'Ang II et sans réaction croisée avec l'Ang III et les peptides de dégradation.

Par ailleurs, le dosage spécifique de l'Ang II présente à ce jour une difficulté méthodologique qui nécessite d'effectuer successivement plusieurs étapes sur l'échantillon sanguin (J. NUSSBERGER et al., Hypertension, 1986, 8, 476-482) :

1. une extraction des différentes angiotensines et des peptides de dégradation pour préparer un extrait plasmatique les contenant ;
2. une séparation par chromatographie liquide à haute performance (HPLC) des différentes angiotensines issues du métabolisme de l'angiotensinogène et présentes dans le plasma ;
3. un dosage radioimmunologique des fractions collectées après élution.

L'extraction des angiotensines du plasma est une étape indispensable commune à toutes les techniques de dosage de l'Ang II ; elle est effectuée par chromatographie sur une colonne de silice selon la méthode décrite par J. NUSSBERGER et al. dans Hypertension, 1985 (7), suppl. 1, I-1-I-7.

A cause de l'étape de séparation par HPLC, cette technique est lourde et longue à mettre en oeuvre ; c'est pourquoi elle ne peut pas être utilisée pour doser un grand nombre d'échantillons plasmatiques dans des conditions de routine.

On a cherché à mettre au point un dosage spécifique de l'Ang II, permettant de mesurer avec précision le taux d'Ang II quelles que soient les concentrations d'Ang I, d'Ang III et des peptides de dégradation présentes dans le milieu et qui soit réalisable en un temps compatible avec une utilisation clinique en routine. Un tel dosage spécifique présente un grand intérêt :

- soit pour étudier la physiologie du système rénine-angiotensine chez différentes espèces animales dans des situations expérimentales,
- soit pour diagnostiquer des situations pathologiques chez l'homme,
- soit pour le diagnostic et le suivi des traitements de l'hypertension artérielle.

Généralement, l'Ang II est dosée à partir du plasma. On peut, selon la présente invention, réaliser le dosage de l'Ang II, soit dans le plasma, soit dans tout autre liquide biologique où elle est présente.

Le dosage selon l'invention peut être effectué pour toute espèce animale chez laquelle on souhaite connaître la mesure spécifique de l'Ang II, et plus particulièrement chez l'homme.

Selon la présente invention, il a été trouvé que l'on peut effectuer un immunodosage spécifique de l'Ang II en utilisant simultanément un anticorps dirigé contre l'Ang II destiné au dosage proprement dit et un anticorps spécifique de l'Ang III ainsi que, éventuellement, un ou plusieurs anticorps spécifiques chacun de l'Ang I ou d'un peptide de dégradation. Par anticorps spécifique de l'Ang III et anticorps spécifique de l'Ang I ou d'un peptide de dégradation, on entend des anticorps présentant chacun une réaction croisée vis-à-vis de l'Ang II, inférieure à 10 %. Ainsi, l'Ang III et éventuellement l'Ang I et les peptides de dégradation, captés chacun par un anticorps spécifique, ne sont pratiquement plus disponibles pour donner des réactions croisées avec l'anticorps anti-Ang II. Le dosage de l'Ang II devient ainsi totalement spécifique, c'est-à-dire totalement exempt de toute interférence due à la présence de peptides apparentés dans l'échantillon.

Selon la présente invention, la mesure de l'Ang II ne comporte que 2 étapes :

1. l'extraction des différentes angiotensines et des peptides de dégradation pour préparer l'extrait de liquide biologique les contenant ;
2. le dosage immunologique proprement dit de l'Ang II contenu dans ledit extrait.

Ainsi, on évite la séparation par HPLC des différentes angiotensines dans l'extrait : cette étape étant la plus lente et la plus laborieuse du protocole de dosage spécifique de l'Ang II.

Le dosage immunologique utilisé est un dosage par compétition : l'Ang II marquée, liée à l'anticorps anti-Ang II est déplacée par l'Ang II non marquée contenue dans l'échantillon à doser. La référence à une courbe d'étalonnage, réalisée avec des étalons contenant des quantités connues d'Ang II, permet de déterminer la concentration en Ang II présente dans l'échantillon.

L'Ang II est marquée, soit par un radioélément, tel que le tritium ($^3$H) ou l'iode 125 ($^{125}$I), soit par une enzyme telle que, par exemple, l'acétylcholinesterase, la peroxydase, la phosphatase alcaline, ou la β-galactosidase, soit par un marqueur fluorescent, soit par un marqueur luminescent.

Selon la présente invention on ajoute dans le milieu réactionnel, un anticorps spécifique de l'Ang III et, éventuellement, un ou plusieurs anticorps spécifiques chacun de l'Ang I ou d'un peptide de dégradation qui masquent l'Ang III et éventuellement l'Ang I et les peptides de dégradation présents dans l'échantillon par formation du (ou des) complexe(s) antigène-anticorps correspondants. On évite ainsi l'apparition de réactions croisées entre l'Ang III, et éventuellement l'Ang I et les peptides de dégradation, présents dans l'échantillon, avec l'anticorps anti-Ang II.

La présente invention a aussi pour objet une trousse pour le dosage spécifique de l'Ang II comprenant :

- un anticorps anti-Ang II,
- une solution d'Ang II marquée,
- des solutions contenant les étalons d'Ang II en concentrations connues et croissantes,
- la ou les solutions de lavage nécessaires,
- une solution d'anticorps anti-Ang III, ledit anticorps présentant une réaction croisée avec l'Ang II, inférieure à 10 %, de préférence inférieure à 5 %,
- et, le cas échéant, une solution d'anticorps anti-Ang I et/ou une solution d'anticorps anti-hexapeptide (IV) et/ou une solution d'anticorps anti-pentapeptide (V), lesdits anticorps présentant une réaction croisée avec l'Ang II, inférieure à 10 %, de préférence inférieure à 5 %.

Lorsque l'Ang II est marquée par une enzyme, la trousse comprend en outre :

- une solution contenant le substrat de ladite enzyme et, le cas échéant, un ou plusieurs réactifs nécessaires pour révéler l'activité de l'enzyme,
- une solution destinée à arrêter la réaction enzymatique.

L'anticorps anti-Ang II utilisé dans la trousse de dosage peut être polyclonal ou monoclonal ; il peut être utilisé en solution ou fixé sur un support solide. Comme supports solides on peut citer les tubes, les plaques de microtitration et les particules, magnétiques ou non.

Selon la présente invention, l'anticorps anti-Ang II préféré est monoclonal ; avantageusement, on le fixe sur un support solide, selon les méthodes bien connues de l'homme du métier.

L'anticorps anti-Ang III peut être un anticorps polyclonal ou un anticorps monoclonal. Il en est de même pour les anticorps anti-Ang I, anti-hexapeptide (IV) et anti-pentapeptide (V).

Lorsque les anticorps utilisés sont en solution, on détermine, préalablement au dosage, la dilution convenable pour que les peptides présents dans l'échantillon à doser soient capturés chacun par l'anticorps anti-peptide correspondant.

La présente invention a également pour objet le procédé utilisant la trousse de dosage selon l'invention. Ledit procédé est caractérisé en ce qu'il comprend les étapes suivantes selon lesquelles :

a) on prépare le milieu réactionnel comprenant :

- l'extrait de liquide biologique à doser,
- un anticorps anti-Ang II,
- de l'Ang II marquée,
- un anticorps anti-Ang III,
- et, le cas échéant, un anticorps anti-Ang I et/ou un anticorps anti-hexapeptide (IV) et/ou un anticorps anti-pentapeptide (V),

b) on prépare simultanément les milieux réactionnels utiles pour l'étalonnage comprenant chacun :

- un étalon d'Ang II,
- un anticorps anti-Ang II,
- de l'Ang II marquée,
- un anticorps anti-Ang III,
- et, le cas échéant, un anticorps anti-Ang I, et/ou un anticorps anti-hexapeptide (IV) et/ou un anticorps anti-pentapeptide (V) ;

c) on laisse incuber à une température comprise entre 4°C et la température ambiante, pendant 12 à 72 heures ;
d) on sépare l'Ang II liée à l'anticorps anti-Ang II de l'Ang II libre ;
e) enfin, on effectue la lecture des résultats selon une méthode appropriée.

Préférentiellement, le procédé selon l'invention est utilisé pour le dosage spécifique de l'Ang II dans un extrait plasmatique.

Lorsque l'anticorps anti-Ang II est utilisé en solution, les étapes a) et b) sont réalisées en mélangeant :

- 25 à 500 µl d'extrait de liquide biologique à doser, ou d'étalon d'Ang II, à un milieu contenant :
- 25 à 500 µl de solution d'anticorps anti-Ang II,
- 25 à 500 µl de solution d'anticorps anti-Ang III,
- le cas échéant; 25 à 500 µl de solution d'anticorps anti-Ang I, et/ou 25 à 500 µl d'anticorps anti-hexapeptide (IV) et/ou 25 à 500 µl d'anticorps anti-pentapeptide (V), et
- 25 à 500 µl de solution d'Ang II marquée.

On peut, éventuellement, effectuer une pré-incubation de 3 à 24 heures avant d'ajouter la solution d'Ang II marquée. L'étape d) est réalisée par un procédé de séparation utilisé classiquement :

- soit par fixation immunologique utilisant des anticorps dirigés contre les immunoglobulines de l'espèce animale à laquelle appartient l'anticorps anti-Ang II utilisé, ces anticorps étant eux-mêmes insolubilisés sur une phase solide (support insoluble tel que par exemple des tubes, des plaques de microtitration ou des particules, magnétiques ou non),
- soit par précipitation physico-chimique du complexe antigène-anticorps à l'aide d'un agent de précipitation, tel que le polyéthylèneglycol,
- soit par séparation de l'antigène libre par le complexe charbon-dextran (J. NUSSBERGER et al., J. Lab. Clin. Med., 1984, 103 (2), 304-312).

Lorsque, suivant un autre mode de réalisation du procédé selon l'invention, l'anticorps anti-Ang II est fixé sur un support solide, la préparation du milieu réactionnel (étapes a et b) est effectuée en présence dudit support. On peut par exemple utiliser des tubes revêtus d'anticorps anti-Ang II dans lesquels on mélange :

- 25 à 500 µl d'extrait de liquide biologique à doser ou d'étalon d'Ang II,
- 25 à 500 µl de solution d'anticorps anti-Ang III,
- le cas échéant, 25 à 500 µl de solution d'anticorps anti-Ang I, et/ou 25 à 500 µl d'anticorps anti-hexapeptide (IV) et/ou 25 à 500 µl d'anticorps anti-pentapeptide (V), et
- 25 à 500 µl de solution d'Ang II marquée.

Selon ce mode de réalisation du procédé de l'invention, la séparation (étape d) est réalisée par aspiration de la solution d'incubation du milieu réactionnel, puis lavage et nouvelle aspiration pour éliminer l'Ang II qui n'est pas complexée par l'anticorps fixée sur le support solide.

On peut également préparer le mélange indiqué ci-dessus dans un tube ou récipient normal et y ajouter l'anticorps anti-Ang II sous la forme de particules, magnétiques ou non, sur lesquelles est fixé l'anticorps anti-Ang II. Dans ce cas, la séparation (étape d) est réalisée selon le même principe que précédemment, mais par application d'un procédé permettant de retenir les particules (centrifugation, filtration, usage d'un aimant s'il s'agit de particules magnétiques).

Dans tous les cas, on effectue ensuite directement l'étape e) de lecture qui s'applique à la phase solide dans laquelle est inclus ou à laquelle est lié l'anticorps anti-Ang II complexant l'Ang II à doser.

D'une façon générale, l'étape de lecture dépend du mode de marquage utilisé pour l'Ang II. Ainsi, on effectue :

- soit une mesure de radioactivité lorsque l'on a utilisé un radiomarquage,
- soit une mesure d'absorbance ou d'émission lumineuse lorsque l'on a utilisé un marquage fluorescent ou luminescent ou un marquage enzymatique selon la nature de l'enzyme et du substrat mis en oeuvre.

Dans la description qui va suivre et dans les revendications, les aminoacides sont nommés en utilisant les abréviations recommandées par l'IUPAC ; de plus, les abréviations suivantes sont utilisées :

BOP :      hexafluorophosphate de benzotriazolyloxy trisdiméthylamino phosphonium
Boc :      tert-butoxycarbonyle
Mob :      para-méthoxybenzyle
DCB :      2,6-dichlorobenzyle
Tos :      tosyle
AcM :      anticorps monoclonal
SAB :      sérum albumine bovine

BGG :        gamma-globuline bovine
cpm :        coup par minute
PEG :        polyéthylèneglycol
LPH :        hémocyanine de Limulus polyphemus
Sulfo MBS :        sel de sodium de N-(3-maléïmidobenzoyloxy) sulfosuccinimide
Bo :        concentration du complexe Ang II marquée-anticorps anti-Ang II, en l'absence d'Ang II, ou d'Ang III, non marquée
B :        concentration du complexe Ang II marquée-anticorps anti-Ang II, pour chaque concentration d'Ang II ou d'Ang III non marquée.

Enfin, un autre objet de la présente invention est la préparation des anticorps anti-Ang III.

L'anticorps anti-Ang III est préparé de façon classique par immunisation d'un animal avec un immunogène. Ledit immunogène est constitué d'un dérivé peptidique fixé sur une protéine modifiée ou protéine vectrice (voir Am. J. Obstet. Gyneco., 1972, 113, p. 751-757). A titre d'exemples d'animaux pouvant être utilisés dans ce procédé, on peut citer le lapin, la chèvre, le mouton, le rat et la souris.

Les anticorps monoclonaux sont obtenus par la méthode de Kohler et Milstein bien connue de l'homme de l'art.

De la même façon, on peut préparer les anticorps anti-Ang I, anti-hexapeptide (IV) et anti-pentapeptide (V).

L'utilisation de protéine vectrice dans l'immunisation vis-à-vis de molécules de faible poids moléculaire (haptène) permet d'induire la formation d'anticorps spécifiques anti-haptènes chez l'animal.

Les protéines vectrices les plus utilisées sont actuellement les albumines d'origine animale (disponibles commercialement, stables, très immunogènes). La synthèse du conjugué protéine-peptide est réalisée par la formation d'une liaison covalente de type thio-éther entre le groupement thiol du peptide et le groupement maléimide de la protéine, introduit au préalable par réaction avec le sulfo MBS.

Selon la présente invention, le dérivé peptidique choisi comme immunogène pour préparer l'anticorps anti-Ang III répond à la formule :

$$\text{H-Arg-Val-Tyr-Ile-His-}R_1\text{-}R_2\text{-Cys-NH}_2 \tag{VI}$$

dans laquelle

- $R_1$ désigne une liaison directe ou un enchaînement comprenant 1 ou 2 acides aminés naturels. Préférentiellement, $R_1$ représente Pro ou Pro lié à un autre acide aminé naturel,
- $R_2$ représente un groupement de formule : $-NH-(CH_2)_n-CO-$ dans lequel n varie entre 1 et 9, préférentiellement n est 5.

Ainsi, selon la présente invention, l'anticorps anti-Ang III préféré est l'antisérum, obtenu par immunisation d'un animal avec l'immunogène constitué du peptide (VI) fixé sur une protéine vectrice.

Plus particulièrement préféré est l'antisérum obtenu par l'immunogène constitué du peptide de formule :

$$\text{H-Arg-Val-Tyr-Ile-His-}R'_1\text{-Ahx-Cys-NH}_2$$

dans laquelle $R'_1$ représente Pro ou Pro lié à un autre acide aminé naturel et Ahx désigne le résidu de l'acide amino-6 hexanoïque, ledit peptide étant porté par la sérumalbumine bovine modifiée par le sulfo MBS. Lorsque l'animal est le lapin, l'antisérum ainsi obtenu présente une réaction croisée avec l'Ang II, inférieure à 1%.

De la même façon, selon la présente invention, le dérivé peptidique choisi comme immunogène pour préparer l'anticorps anti-Ang I répond à la formule :

$$\text{H-Cys-}R_2\text{-}R_3\text{-Arg-Val-Tyr-Ile-His-Pro-Phe-His-Leu-OH} \tag{VII}$$

dans laquelle

- $R_3$ désigne une liaison directe ou un enchaînement comprenant 1 ou 2 acides aminés naturels. Préférentiellement, $R_3$ représente Asp ou Asp lié à un autre acide aminé naturel,
- $R_2$ a la valeur indiquée ci-dessus pour (VI).

Selon la présente invention, le dérivé peptidique choisi comme immunogène pour préparer l'anticorps anti-hexapeptide répond à la formule :

$$\text{H-Val-Tyr-Ile-His-}R_1\text{-}R_2\text{-Cys-NH}_2 \tag{VIII}$$

dans laquelle $R_1$ et $R_2$ ont les significations indiquées ci-dessus pour (VI).

Enfin, selon la présente invention, le dérivé peptidique choisi comme immunogène pour préparer l'anticorps anti-pentapeptide répond à la formule :

$$\text{H-Tyr-Ile-His-}R_1\text{-}R_2\text{-Cys-NH}_2 \tag{IX}$$

dans laquelle $R_1$ et $R_2$ ont les significations indiquées ci-dessus pour (VI).

Dans les définitions de $R_1$, $R'_1$ et $R_3$, l'autre acide aminé naturel peut être choisi parmi les acides aminés ci-après : Ala, Val, Leu, Ile, Pro, Phe, Trp, Met, Ser, Thr, Cys, Tyr.

Ainsi, selon la présente invention, les anticorps sont obtenus par immunisation d'un animal avec un dérivé peptidique de formule VII ou un dérivé peptidique de formule :

$$\text{H-}X_1\text{-Tyr-Ile-His-}R_1\text{-}R_2\text{-Cys-NH}_2$$

dans laquelle

- $R_1$ désigne une liaison directe ou un enchaînement comprenant 1 ou 2 acides aminés naturels. Préférentiellement, $R_1$ représente Pro ou Pro lié à un autre acide aminé naturel,
- $R_2$ représente un groupement de formule $\text{-NH-(CH}_2)_n\text{-CO-}$ dans lequel n varie entre 1 et 9, préférentiellement n est 5, et caractérisé en ce que :

lorsque $X_1$ représente une liaison directe, l'anticorps est spécifique du pentapeptide,
lorsque $X_1$ représente le résidu de la valine, l'anticorps est spécifique de l'hexapeptide,
lorsque $X_1$ représente le résidu du dipeptide Arg-Val, l'anticorps est spécifique de l'angiotensine III.

Les anticorps selon l'invention présentent une réaction croisée avec l'Ang II inférieure à 10 %, de préférence inférieure à 5 %.

Les dérivés peptidiques ci-dessus mis en oeuvre pour l'obtention des anticorps selon l'invention constituent un autre objet de l'invention. Ces peptides sont obtenus selon les méthodes habituelles de la chimie des peptides. En particulier, ils peuvent être préparés par un procédé étape par étape dit "procédé Stepwise", à partir du C terminal. On réalise de préférence la synthèse de ces peptides en phase solide.

L'invention va être maintenant décrite plus en détail par les exemples illustratifs ci-après.

Exemple 1

Préparation de l'anti-Ang III.

A) Préparation du peptide (VI) dans lequel $R_1$=Pro et $R_2$=Ahx.

Le composé (VI) a été synthétisé en phase solide en utilisant les procédés connus en chimie des peptides.

La phase solide utilisée est la résine méthyl-4 benzydrylamine fonctionnalisée à un taux de 0,4 mmole par gramme. La condensation successive des aminoacides est effectuée par le réactif BOP, les fonctions alpha aminées sont protégées de façon temporaire par un groupement Boc et déprotégées par une solution acide trifluoroacétique/dichloroéthane/éthanedithiol (35/70/5 ; v/v/v). Les chaînes latérales des différents aminoacides sont protégées par des groupements convenables : Mob pour Cys, Boc pour His, DCB pour Tyr, Tos pour Arg.

Une fois synthétisé, le peptide est déprotégé et séparé de la résine par action de l'acide fluorhydrique contenant 10 % d'anisole pendant 1 heure à 0°C. Le peptide est ensuite précipité à l'éther, repris par une solution acétonitrile/eau/acide trifluoro-acétique (60/40/0,1 ; v/v/v) et lyophilisé. La purification est effectuée par chromatographie liquide sous pression sur colonne de phase inverse.

Les différents résultats analytiques sont en conformité avec la structure attendue pour le peptide (VI).

B) Préparation de la protéine modifiée (LERNER, R.A. et al. (1981) Proc. Nat. Acad. Sci. USA, 78, 3403-3407).

La protéine choisie pour fixer le peptide (VI) est la sérumalbumine bovine (SAB) modifiée par réaction avec le sulfo MBS.

A une solution de 4 ml de SAB (à 10 mg/ml dans le tampon phosphate de potassium 0,05 M pH 8,0 sont additionnés 22,4 mg de sulfo MBS (Pierce) solide, soit une stoechiométrie de 80 équivalents MBS/SAB. On laisse la réaction évoluer 1 heure à température ambiante sous agitation douce, puis on dialyse le mélange 18 heures à 4°C contre 2 litres de tampon phosphate 0,1 M pH 7,0.

La détermination du taux de substitution est réalisée par la mesure du nombre de groupements maléimide introduits grâce à la réaction à la cystéine radiomarquée par le [14]C. A 0,4 ml de milieu réactionnel on ajoute 0,6 ml de tampon phosphate 0,1 M pH 7,0 et 0,05 ml de cystéine 20 mM additionnée de cystéine $^{14}$C (concentration finale 3 KBq/umole de cystéine). Après incubation 1 heure 30 à 30°C, puis filtration du milieu réactionnel sur une colonne de chromatographie dans des conditions convenables, la mesure de la radioactivité permet de calculer le taux de modification observé qui est de 11 groupements maléimide par mole de SAB.

C) Couplage du peptide (VI).

5 mg de peptide (VI) préparé ci-dessus sont dissous dans 0,25 ml d'eau distillée. Cette solution est additionnée à 1 ml de solution de SAB modifiée (10 mg/ml). Le mélange est incubé 3 heures à température ambiante et 18 heures à 4°C, puis on effectue plusieurs dialyses extensives contre du tampon phosphate 0,1 M pH 7,0.

Le test à la cystéine radiomarquée est réalisé à nouveau sur la protéine après couplage du peptide. La détermination du nombre de groupements maléimide non modifiés indique par différence un taux de substitution de 7 moles de peptide (VI) par mole de SAB.

Deux autres conjugués immunogènes utilisant de l'hémocyanine de Limulus polyphemus (LPH) et de la transferrine humaine (SIGMA) comme protéines porteuses ont été préparés dans les mêmes conditions.

D) Préparation de l'anticorps (ou antisérum) anti-Ang III.

L'animal utilisé est le lapin de race néozélandaise. La première immunisation consiste en l'injection de 1 mg d'immunogène en présence d'adjuvant complet de Freund par voie intradermique. Les rappels sont effectués à intervalle de 1 mois avec la même quantité d'immunogène en présence d'adjuvant complet de Freund par voie sous-cutanée.

E) Détermination du titre des sérums anti-Ang III.

100 μl d'une dilution de sérum de lapin en tampon imidazole-HCl 0,1 M pH 7,4 + SAB 0,2 % sont incubés en présence de 100 μl d'Ang III marquée à l'iode 125 ayant une activité spécifique de 74 TBq/mmole (Amersham), apportant 5 000 cpm.

La séparation de l'Ang III liée à l'anticorps de l'Ang III libre est réalisée par l'addition de 1 ml de polyéthylèneglycol 6 000 (PEG) à 20 % et de 100 μl de solution de globulines bovines à 10 mg/ml. Après centrifugation, la radioactivité du culot correspondant à l'Ang III liée à l'anticorps est mesurée. La technique utilisée est celle décrite par GREENWOOD, F.C. et al. (Biochem. J., 1963, 89, 114-118).

Le sérum de lapin lie au maximum 60 de l'Ang III marquée à l'iode 125.

F) Caractérisation immunologique de l'antisérum anti-Ang III.

a) Evaluation de l'immunoréactivité de l'antisérum préparé vis-à-vis de l'Ang III.

100 μl d'une solution diluée de l'antisérum anti-Ang III (correspondant à environ 50 % de liaison à l'Ang III iodée) sont incubés en présence de 50 μl d'Ang III libre et 50 μl d'Ang III iodée apportant 5 000 cpm pendant 18 heures à 4°C. La séparation Ang III liée et Ang III libre se fait par précipitation au polyéthylèneglycol. La liaison Ang III iodée-anticorps est déplacée de 50 % par l'addition de 250 pg d'Ang III. L'anticorps reconnaît donc l'Ang III.

b) Réactions croisées de l'anticorps anti-Ang III avec l'Ang I et l'Ang II.

La réaction croisée des anticorps anti-Ang III a été mesurée vis-à-vis de l'Ang I et de l'Ang II. Le pourcentage est inférieur à 1 % pour les deux peptides.

L'anticorps anti-Ang III peut être utilisé tel qu'il est obtenu dans l'antisérum ou après purification sur protéine A-Sepharose.

Exemple 2

Capacité de capture de l'Ang III par les anticorps anti-Ang III décrits ci-dessus en présence d'anticorps monoclonal anti-Ang II 4D8 et d'Ang II iodée.

L'anticorps 4D8 est une immunoglobuline de souris ; il est décrit dans les actes du Congrès "The Renin Angiotensin System as a Therapeutic Target", Bâle, 29-31 octobre 1989, communication SANOFI.

On prépare une solution contenant :

- 100 μl d'une solution d'anticorps de lapin anti-Ang III, obtenu à l'exemple 1,
- 50 μl d'une solution d'Ang III,
- 50 μl d'une solution d'Ang II iodée (activité spécifique de 74 TBq/mmole (Amersham), apportant 4 000 cpm,
- 100 μl d'une solution d'anticorps anti-Ang II (4D8), dilué de telle façon qu'il ne lie que 30 % de l'Ang II iodée.

Le volume de réaction est complété à 400 μl avec le tampon d'incubation, soit imidazole-HCl (0,1 M ; pH 7,4) contenant 0,2 % de SAB. L'incubation est réalisée pendant 18 heures à + 4°C.

On ajoute une suspension de 500 μl de particules magnétiques recouvertes d'anticorps dirigés contre les immunoglobulines de souris (Amerlex M, commercialisé par Amersham). L'incubation est réalisée pendant 15 minutes, puis on sépare en utilisant un aimant les particules magnétiques-du-milieu réactionnel et on mesure la radioactivité contenue dans la phase magnétique.

On a réalisé une gamme de mesures en faisant varier la quantité d'Ang III de 0 à 40 pg/essai. Des essais de contrôle ont été effectués en l'absence d'anticorps anti-Ang III.

La mesure de la radioactivité permet d'exprimer les résultats par le rapport des concentrations B/Bo du complexe Ang II iodée-anticorps anti-Ang II pour chaque quantité d'Ang III.

Ces résultats sont rapportés dans le tableau 1.

TABLEAU 1

| Ang III (pg/essai) | B/Bo (en pour-cent) | |
|---|---|---|
| | en absence d'anticorps anti-Ang III | en présence d'anticorps anti-Ang III |
| 0 | 100 | 100 |
| 5 | 67 | 100 |
| 10 | 39 | 100 |
| 20 | 18 | 100 |
| 40 | 5 | 96 |

Comme le montrent les résultats du tableau 1, la liaison de l'Ang II iodée à l'anticorps 4D8 est diminuée par l'addition d'Ang III ; cette liaison est restaurée intégralement par la présence d'anticorps anti-Ang III.

Dans cette expérience, il a en outre été vérifié que l'addition au milieu réactionnel d'anticorps anti-Ang III à la concentration utilisée n'a pas d'influence sur la réaction de l'Ang II avec les anticorps anti-Ang II.

Exemple 3

Dosage spécifique de l'Ang II en présence d'anticorps anti-Ang III :

Selon l'un des modes de réalisation décrits plus haut, la trousse utilisée dans cet exemple comprend les anticorps monoclonaux anti-Ang II fixés sur un support solide.

Plus précisément, les-anticorps sont fixés sur les parois d'un tube (Startube® commercialisé par NUNC). La trousse est alors constituée des éléments suivants :

- Startube NUNC revêtu des anticorps monoclonaux anti-Ang II 4D8 de telle façon que 30 % de l'Ang II iodée soient liés,
- des solutions d'Ang II, pour l'étalonnage, calibrées par rapport au standard international Ang II lot 86/538, fourni par le Medical Research Council,
- une solution d'Ang II marquée à l'iode 125, d'activité telle que définie à l'exemple 2,
- une solution de l'anticorps polyclonal de lapin anti-Ang III, préparé à l'exemple 1 ci-dessus et dilué de façon telle qu'il permette de capter au moins 10 pg d'Ang III par essai.

Le dosage est réalisé en tampon imidazole-HCl (0,1 M, pH 7,4) contenant 0,2 % de SAB. Le volume final de la réaction est de 250 $\mu$l.

a) Courbe d'étalonnage en présence de 10 pg d'Ang III.

Dans le tube revêtu d'anticorps monoclonal 4D8, 100 $\mu$l d'anticorps polyclonal de lapin anti-Ang III sont incubés avec 50 $\mu$l d'une solution d'Ang III (correspondant à 10 pg d'Ang III), 50 $\mu$l d'une solution d'Ang II étalon et 50 $\mu$l d'une solution d'Ang II iodée (4 000 cpm), pendant 18 heures à + 4°C.

La radioactivité est mesurée dans les tubes secs après aspiration du milieu d'incubation et lavage avec 1 ml de tampon imidazole-HCl (0,1 M, pH 7,4). La radioactivité est celle du complexe anticorps anti-Ang II-Ang II iodée.

On a réalisé une gamme d'étalonnage en faisant varier les quantités d'Ang II de 0 à 40 pg/essai. La mesure de radioactivité permet d'exprimer les résultats par le rapport de concentration B/Bo du complexe Ang II iodée - anticorps anti-Ang II pour chaque quantité d'Ang II (colonne 1).

On ajoute 10 pg d'Ang III dans chacun des essais correspondant à la courbe d'étalonnage (colonne 2) et l'on observe l'abaissement du rapport B/Bo dû à la réaction croisée de l'anticorps anti-Ang II avec l'Ang III présente.

Enfin, dans une troisième série d'expériences (colonne 3) on ajoute 10 pg d'Ang III et de l'anticorps anti-Ang III dans chacun des essais correspondant à la courbe d'étalonnage.

Les résultats sont rapportés dans le tableau 2 ci-après.

TABLEAU 2

| Ang II (pg/essai) | colonne 1 B/Bo (pour-cent) | Surchage 10 pg Ang III B/Bo (pour-cent) | |
|---|---|---|---|
| | | colonne 2 en absence d'anticorps anti-Ang III | colonne 3 en présence d'anticorps anti-Ang III |
| 0 | 100 | 23 | 100 |
| 1,25 | 84 | 21 | 86 |
| 2,5 | 81 | 22 | 83 |
| 5 | 67 | 22 | 66 |
| 10 | 47 | 18 | 49 |
| 20 | 29 | 12 | 36 |
| 40 | 17 | 11 | 21 |

On observe que la présence d'anticorps anti-Ang III permet de retrouver les valeurs de la courbe d'étalonnage grâce à la capture spécifique de l'Ang III dans le milieu réactionnel.

On a par ailleurs vérifié, comme à l'exemple 2, que l'ajout d'anticorps anti-Ang III, à la concentration utilisée, n'a pas d'influence sur la courbe d'étalonnage de l'Ang II.

b) Dosage spécifique de l'Ang II à partir du plasma.

Le dosage proprement dit est effectué sur un extrait plasmatique humain, extrait obtenu selon la technique décrite par J. NUSSBERGER et al. dans Hypertension, 1985 (référence citée), puis concentré.

Dans le tube revêtu d'anticorps monoclonal 4D8, 100 µl d'anticorps polyclonal de lapin anti-Ang III sont incubés avec 100 µl de solution d'Ang II étalon ou d'extrait plasmatique à doser et 50 ul d'une solution d'Ang.II iodée (4 000 cpm), pendant 18 heures à + 4°C.

La radioactivité est mesurée dans les tubes secs après aspiration du milieu d'incubation, comme à l'exemple 3 a).

Une détermination de l'Ang II effectuée en l'absence d'anticorps anti-Ang III, conduit à déterminer une quantité apparente de 81 pg dans l'essai, tandis que, en présence d'anticorps anti-Ang III, on ne mesure que 57 pg dans l'essai. La différence résulte de l'élimination de la quantité d'Ang III immunocapturée par les anticorps anti-Ang III.

On en déduit que la présence d'Ang III conduit dans ce cas à une erreur de : $\frac{81-57}{57}$, soit 42 % sur la détermination directe de l'Ang II réalisée à l'aide d'anticorps anti-Ang II (4D8), en raison de la réaction croisée entre cet anticorps et l'Ang III. Cette erreur peut être supprimée grâce à l'emploi simultané de l'anticorps anti-Ang III qui est l'un des objets de la présente invention.

Exemple 4

Préparation de l'anticorps anti-Ang I.
On prépare le peptide (VII) dans lequel $R_2$=Ahx et $R_3$=Asp, selon le mode opératoire décrit à l'exemple 1, puis on prépare l'antisérum de lapin par immunisation selon la méthode décrite à l'exemple 1.

Exemple 5

Préparation de l'anticorps anti-hexapeptide.
On prépare le peptide (VIII) das lequel $R_1$=Pro et $R_2$=Ahx en utilisant le procédé décrit à l'exemple 1, étape A.
On prépare ensuite l'anticorps (antisérum de lapin) en utilisant la méthode décrite à l'exemple 1, étapes B, C, D, puis on le caractérise.

Exemple 6

Préparation de l'anticorps anti-pentapeptide.
On procède comme à l'exemple précédent pour préparer le peptide (IX) dans lequel $R_1$=Pro et $R_2$=Ahx, puis on prépare l'anti-sérum de lapin par immunisation, en utilisant la méthode décrite à l'exemple 1, puis on le caractérise.

Exemple 7

Capacité de capture du pentapeptide (V) par les anticorps anti-pentapeptide préparés à l'exemple 4, en présence d'anti-Ang II 4D8 et d'Ang II iodée.

On prépare une solution contenant :

- 100 μl d'une solution d'anticorps anti-pentapeptide préparé à l'exemple 5,
- 100 μl d'une solution de pentapeptide,
- 50 μl d'une solution d'Ang II iodée (activité spécifique de 74 TBq/mmole (Amersham), apportant 4 000 cpm et on la place dans un tube revêtu d'anticorps selon l'exemple 3.

La dilution finale de l'anticorps anti-pentapeptide est 1/25. L'incubation est réalisée pendant 18 heures à 4°C. La radioactivité est mesurée dans les tubes secs après aspiration du milieu d'incubation et lavage avec 1 ml de tampon imidazole-HCl (0,1 M, pH 7,4). La radioactivité est celle du complexe anticorps anti-Ang II-Ang II iodée.

On procède ensuite comme à l'exemple 2 et l'on mesure les résultats rapportés dans le tableau 3 ci-après :

TABLEAU 3

| Pentapeptide (pg/essai) | B/Bo (en pour-cent) | |
|---|---|---|
| | en absence d'anticorps anti-pentapeptide | en présence d'anticorps anti-pentapeptide |
| 0 | 100 | 100 |
| 1,25 | 95 | 100 |
| 2,5 | 92 | 100 |
| 5 | 75 | 100 |
| 10 | 69 | 100 |
| 20 | 48 | 97 |

Exemple 8

Dosage spécifique de l'Ang II en présence d'anticorps anti-Ang II et d'anticorps anti-pentapeptide (V).

Selon l'un des modes de réalisation décrits plus haut, la trousse utilisée dans cet exemple comprend les anticorps monoclonaux anti-Ang II fixés sur un support solide.

Plus précisément, les anticorps sont fixés sur les parois d'un tube (Startube® commercialisé par NUNC). La trousse est alors constituée des éléments suivants :

- Startube NUNC revêtu des anticorps monoclonaux anti-Ang II 4D8 de telle façon que 30 % de l'Ang II iodée soient liés,
- des solutions d'Ang II, pour l'étalonnage, calibrées par rapport au standard international Ang II lot 86/538, fourni par le Medical Research Council,
- une solution d'Ang II marquée à l'iode 125, d'activité telle que définie à l'exemple 2,
- une solution de l'anticorps polyclonal de lapin anti-Ang III, préparé à l'exemple 1 ci-dessus et dilué de façon telle qu'il permette de capter au moins 10 pg d'Ang III par essai,
- une solution de l'anticorps polyclonal de lapin anti-pentapeptide (V) préparé à l'exemple 5 ci-dessus de telle façon qu'il permette de capter au moins 10 pg de pentapeptide par essai.

Le dosage est réalisé en tampon imidazole-HCl (0,1 M, pH 7,4) contenant 0,2 % de SAB. Le volume final de la réaction est de 250 μl.

On a effectué la courbe d'étalonnage en présence de 4 pg d'Ang III et 4 pg de pentapeptide.

Dans le tube revêtu d'anticorps monoclonal 4D8, 100 μl d'une solution d'anticorps polyclonal de lapin anti-Ang III et anti-pentapeptide sont incubés avec 50 μl d'une solution d'Ang III et de pentapeptide (V) (correspondant à 4 pg de chacun des peptides), 50 μl d'une solution d'Ang II étalon et 50 μl d'une solution d'Ang II iodée (4 000 cpm), pendant 18 heures à + 4°C.

La radioactivité est mesurée dans les tubes secs après aspiration du milieu d'incubation et lavage avec 1 ml de tampon imidazole-HCl (0,1 M, pH 7,4). La radioactivité est celle du complexe anticorps anti-Ang II-Ang II iodée.

On a réalisé une gamme d'étalonnage en faisant varier les quantités d'Ang II de 0 à 40 pg/essai. La mesure de radioactivité permet d'exprimer les résultats par le rapport de concentration B/Bo du complexe Ang II iodée - anticorps anti-Ang II pour chaque quantité d'Ang II (colonne 1).

On ajoute 4 pg d'Ang III et 4 pg de pentapeptide (V) dans chacun des essais correspondant à la courbe d'étalonnage (colonne 2) et l'on observe l'abaissement du rapport B/Bo dû à la réaction croisée de l'anticorps anti-Ang II avec l'Ang III et le pentapeptide (V) présents.

Enfin, dans une troisième série d'expériences (colonne 3) on ajoute 4 pg d'Ang III, 4 pg de pentapeptide et les anticorps anti-Ang III et anti-pentapeptide (V) dans chacun des essais correspondant à la courbe d'étalonnage.

Les résultats sont rapportés dans le tableau 4 ci-après :

TABLEAU 4

| Ang II (pg/essai) | colonne 1 B/Bo (pour-cent) | Surchage 4 pg Ang III 4 pg Ang 4-8 B/Bo (pour-cent) | |
|---|---|---|---|
| | | colonne 2 en absence d'anticorps anti-Ang III et anti-pentapeptide | colonne 3 en présence d'anticorps anti-Ang III et anti-pentapeptide |
| 0 | 100 | 53 | 100 |
| 2,5 | 85 | 47 | 87 |
| 5 | 72 | 44 | 73 |
| 10 | 56 | 34 | 55 |
| 20 | 38 | 27 | 38 |
| 40 | 22 | 19 | 25 |

On observe que la présence d'anticorps anti-Ang III et anti-pentapeptide (V) permet de retrouver les valeurs de la courbe d'étalonnage grâce à la capture spécifique de l'Ang III et du pentapeptide (V) dans le milieu réactionnel.

On a par ailleurs vérifié, comme à l'exemple 2, que l'ajout d'anticorps anti-Ang III et anti-pentapeptide (V) à la concentration utilisée, n'a pas d'influence sur la courbe d'étalonnage de l'Ang II.

## Revendications

1. Une trousse pour le dosage spécifique de l'angiotensine II comprenant :

   - un anticorps anti-Ang II,
   - une solution d'Ang II marquée,
   - des solutions contenant des étalons d'Ang II en concentrations connues et croissantes,
   - la ou les solutions de lavage nécessaires,
   - une solution d'anticorps anti-Ang III, ledit anticorps présentant une réaction croisée avec l'Ang II, inférieure à 10 %, de préférence inférieure à 5 %,
   - et, éventuellement, une solution d'anticorps anti-Ang I et/ou une solution d'anticorps anti-pentapeptide et/ou une solution d'anticorps anti-hexapeptide, lesdits anticorps présentant une réaction croisée avec l'Ang II inférieure à 10 %, de préférence inférieure à 5 %.

2. Trousse selon la revendication 1, caractérisée en ce que l'Ang II est marquée par une enzyme.

3. Trousse selon la revendication 2, caractérisée en ce qu'elle comprend en outre une solution contenant le substrat de l'enzyme marquant l'Ang II, le cas échéant, un ou plusieurs réactifs pour révéler l'activité de l'enzyme et une solution destinée à arrêter la réaction enzymatique.

4. Trousse selon la revendication 1, caractérisée en ce que l'Ang II est marquée soit par un composé fluorescent, soit par un composé luminescent, soit par un radioélément.

5. Trousse selon l'une quelconque des revendications 1 à 4, dans laquelle l'anticorps anti-Ang II, les anticorps anti-Ang III, et anti-Ang I, anti-pentapeptide et anti-hexapeptide sont des anticorps monoclonaux ou polyclonaux.

6. Trousse selon l'une quelconque des revendications 1 à 5, caractérisée en ce que l'anticorps anti-Ang II est en solution ou est fixé sur un support solide.

7. Trousse selon la revendication 6, caractérisée en ce que le support solide est un tube, une plaque de microtitration ou des particules, magnétiques ou non.

8. Trousse selon la revendication 6, caractérisée en ce que les anticorps sont polyclonaux et proviennent chacun d'un antisérum de lapin.

9. Anticorps obtenu par immunisation d'un animal avec un dérivé peptidique de formule :
$$H\text{-}X_1\text{-}Tyr\text{-}Ile\text{-}His\text{-}R_1\text{-}R_2\text{-}Cys\text{-}NH_2$$
   dans laquelle

   - $R_1$ désigne une liaison directe ou un enchaînement comprenant 1 ou 2 acides aminés naturels,
   - $R_2$ représente un groupement de formule : $\text{-}NH\text{-}(CH_2)_n\text{-}CO\text{-}$dans lequel n varie entre 1 et 9,
   - $X_1$ représente une liaison directe, Val ou Arg-Val, selon la spécificité de l'antisérum à préparer,

   ledit dérivé peptidique étant fixé sur une protéine vectrice, ledit anticorps présentant une réaction croisée avec l'angiotensine II, inférieure à 10 %, de préférence inférieure à 5 %.

10. Anticorps anti-Ang III selon la revendication 9, caractérisé en ce que le dérivé peptidique est de formule :
$$H\text{-}Arg\text{-}Val\text{-}Tyr\text{-}Ile\text{-}His\text{-}R_1\text{-}R_2\text{-}Cys\text{-}NH_2 \qquad\qquad (VI)$$
    dans laquelle

- $R_1$ désigne une liaison directe ou un enchaînement comprenant 1 ou 2 acides aminés naturels,
- $R_2$ représente un groupement de formule : $-NH-(CH_2)_n-CO-$ dans lequel n varie entre 1 et 9.

11. Anticorps anti-Ang III selon l'une des revendications 9 ou 10, caractérisé en ce que le dérivé peptidique répond à la formule :

$$H-Arg-Val-Tyr-Ile-His-R'_1-Ahx-Cys-NH_2$$

dans laquelle

- Ahx désigne le résidu de l'acide amino-6 hexanoïque,
- et $R'_1$ représente Pro ou Pro lié à un autre acide aminé naturel.

12. Anticorps anti-Ang III selon la revendication 11, caractérisé en ce qu'il est un antisérum de lapin et en ce qu'il présente une réaction croisée avec l'Ang II, inférieure à 1 %.

13. Anticorps anti-pentapeptide (V) selon la revendication 9, caractérisé en ce que le dérivé peptidique est de formule :

$$H-Tyr-Ile-His-R_1-R_2-Cys-NH_2 \qquad (IX)$$

dans laquelle

- $R_1$ désigne une liaison directe ou un enchaînement comprenant 1 ou 2 acides aminés naturels,
- $R_2$ représente un groupement de formule : $-NH-(CH_2)_n-CO-$ dans lequel n varie entre 1 et 9.

14. Anticorps anti-pentapeptide (V) selon la revendication 13, caractérisé en ce que le dérivé peptidique répond à la formule :

$$H-Tyr-Ile-His-R'_1-Ahx-Cys-NH_2$$

dans laquelle

- Ahx désigne le résidu de l'acide amino-6 hexanoïque,
- et $R'_1$ représente Pro ou Pro lié à un autre acide aminé naturel.

15. Anticorps anti-hexapeptide (IV) selon la revendication 9, caractérisé en ce que le dérivé peptidique est de formule :

$$H-Val-Tyr-Ile-His-R_1-R_2-Cys-NH_2 \qquad (VIII)$$

dans laquelle

- $R_1$ désigne une liaison directe ou un enchaînement comprenant 1 ou 2 acides aminés naturels,
- $R_2$ représente un groupement de formule : $-NH-(CH_2)_n-CO-$ dans lequel n varie entre 1 et 9.

16. Anticorps anti-hexapeptide (IV) selon la revendication 15; caractérisé en ce que le dérivé peptidique répond à la formule :

$$H-Val-Tyr-Ile-His-R'_1-Ahx-Cys-NH_2$$

dans laquelle

- Ahx désigne le résidu de l'acide amino-6 hexanoïque,
- et $R'_1$ représente Pro ou Pro lié à un autre acide aminé naturel.

17. Anticorps anti-Ang I, caractérisé en ce qu'il est obtenu par immunisation d'un animal avec un dérivé peptidique de formule :

$$H-Cys-R_2-R_3-Arg-Val-Tyr-Ile-His-Pro-Phe-His-Leu-OH \qquad (VII)$$

dans laquelle

- $R_3$ désigne une liaison directe ou un enchaînement comprenant 1 ou 2 acides aminés naturels,
- $R_2$ représente un groupement de formule : $-NH-(CH_2)_n-CO-$ dans lequel n varie entre 1 et 9.

18. Anticorps selon la revendication 17, caractérisé en ce que le dérivé peptidique répond à la formule :

$$H-Cys-Ahx-R'_3-Arg-Val-Tyr-Ile-His-Pro-Phe-His-Leu-OH$$

dans laquelle

- Ahx désigne le résidu de l'acide amino-6 hexanoïque,
- R'$_3$ représente Asp ou Asp lié à un autre acide aminé naturel.

19. Procédé pour le dosage de l'angiotensine II, utilisant la trousse de dosage selon la revendication 1, caractérisé en ce qu'il comprend les étapes suivantes selon lesquelles :

   a) on prépare le milieu réactionnel comprenant :

   - l'extrait de liquide biologique à doser,
   - un anticorps anti-Ang II,
   - de l'Ang II marquée,
   - un anticorps anti-Ang III,
   - et, éventuellement, un anticorps anti-Ang I et/ou un anticorps anti-pentapeptide et/ou un anticorps anti-hexapeptide ;

   b) on prépare simultanément les milieux réactionnels utiles pour l'étalonnage comprenant chacun :

   - un étalon d'Ang II,
   - un anticorps anti-Ang II,
   - de l'Ang II marquée,
   - un anticorps anti-Ang III,
   - et, éventuellement, un anticorps anti-Ang I et/ou un anticorps anti-pentapeptide et/ou un anticorps anti-hexapeptide ;

   c) on laisse incuber à une température comprise entre 4°C et la température ambiante, pendant 12 à 72 heures ;
   d) on sépare l'Ang II liée à l'anticorps anti-Ang II de l'Ang II libre ;
   e) enfin, on effectue la lecture des résultats selon une méthode appropriée.

20. Procédé selon la revendication 19, caractérisé en ce que les étapes a) et b) sont réalisées en mélangeant ;

   - 25 à 500 µl d'extrait de liquide biologique à doser, ou d'étalon d'Ang II, à un milieu contenant :
   - 25 à 500 µl de solution d'anticorps anti-Ang II,
   - 25 à 500 µl de solution d'anticorps anti-Ang III,
   - éventuellement 25 à 500 µl de solution d'anticorps anti-Ang I et/ou 25 à 500 µl de solution d'anticorps anti-pentapeptide et/ou 25 à 500 µl de solution d'anticorps anti-hexapeptide, et
   - 25 à 500 µl de solution d'Ang II marquée.

21. Procédé selon la revendication 20, caractérisé en ce que les étapes a) et b) sont réalisées en mélangeant :

   - 25 à 500 µl d'extrait de liquide biologique à doser, ou d'étalon d'Ang II, à un milieu contenant :
   - 25 à 500 µl de solution d'anticorps anti-Ang II,
   - 25 à 500 µl de solution d'anticorps anti-Ang III, et
   - 25 à 500 µl de solution d'Ang II marquée.

22. Procédé selon la revendication 19, caractérisé en ce que les étapes a) et b) sont réalisées en utilisant des tubes revêtus d'anticorps anti-Ang II dans lesquels on mélange :

   - 25 à 500 µl d'extrait de liquide biologique à doser ou d'étalon d'Ang II, à un milieu contenant :
   - 25 à 500 µl de solution d'anticorps anti-Ang III,
   - 25 à 500 µl de solution d'Ang II marquée,

   et l'étape d) est réalisée par aspiration de la solution d'incubation du milieu réactionnel, puis lavage et nouvelle aspiration pour éliminer l'Ang II qui n'est pas complexée par l'anticorps fixé sur le support solide.

23. Procédé selon la revendication 22, caractérisé en ce que l'anticorps anti-Ang II est fixé sur des particules, magnétiques ou non.

**24.** Procédé selon la revendication 23, caractérisé en ce qu'il est utilisé pour le dosage d'un extrait plasmatique.

**25.** Dérivé peptidique de formule :

$$\text{H-X}_1\text{-Tyr-Ile-His-R}_1\text{-R}_2\text{-Cys-NH}_2$$

dans laquelle

- R$_1$ désigne une liaison directe ou Un enchaînement comprenant 1 ou 2 acides aminés naturels, préférentiellement, R$_1$ représente Pro ou Pro lié à un autre acide aminé naturel,
- R$_2$ représente un groupement de formule : -NH-(CH$_2$)$_n$-CO-dans lequel n varie entre 1 et 9, préférentiellement n est 5,
- X$_1$ représente une liaison directe, le résidu de la valine ou le résidu du dipeptide Arg-Val.

**26.** Dérivé peptidique de formule :

$$\text{H-Cys-R}_2\text{-R}_3\text{-Arg-Val-Tyr-Ile-His-Pro-Phe-His-Leu-OH (VII)}$$

dans laquelle

- R$_3$ désigne une liaison directe ou un enchaînement comprenant 1 ou 2 acides aminés naturels, préférentiellement, R$_3$ représente Asp ou Asp lié à un autre acide aminé naturel,
- R$_2$ représente un groupement de formule : -NH-(CH$_2$)$_n$-CO-dans lequel n varie entre 1 et 9, préférentiellement n est 5.

**Patentansprüche**

1. Kit zur spezifischen Bestimmung von Angiotensin II, welcher umfaßt:

   - einen anti-Ang II-Antikörper,
   - eine Lösung von markiertem Ang II,
   - Lösungen, die Ang II-Standards in bekannten und zunehmenden Konzentrationen enthalten,
   - die erforderliche Waschlösung oder die erforderlichen Waschlösungen,
   - eine Lösung von anti-Ang III-Antikörper, welcher Antikörper eine Kreuzreaktion mit Ang II von weniger als 10 %, vorzugsweise weniger als 5 % aufweist,
   - und gegebenenfalls eine Lösung von anti-Ang I-Antikörper und/oder eine Lösung von anti-Pentapeptid-Antikörper und/oder eine Lösung von anti-Hexapeptid-Antikörper, welche Antikörper eine Kreuzreaktion mit Ang II von weniger als 10 %, vorzugsweise weniger als 5 % aufweisen.

2. Kit nach Anspruch 1, dadurch gekennzeichnet, daß Ang II mit einem Enzym markiert ist.

3. Kit nach Anspruch 2, dadurch gekennzeichnet, daß er außerdem eine Lösung umfaßt, die das Substrat des Ang II markierenden Enzyms, gegebenenfalls ein oder mehrere Reagentien zur Darstellung der Aktivität des Enzyms und eine Lösung zum Anhalten der enzymatischen Reaktion enthält.

4. Kit nach Anspruch 1, dadurch gekennzeichnet, daß Ang II entweder mit einer fluoreszierenden Verbindung, mit einer lumineszenten Verbindung oder einem radioaktiven Element markiert ist.

5. Kit nach einem der Ansprüche 1 bis 4, bei welchem der anti-Ang II-Antikörper, die anti-Ang III- und anti-Ang I-, anti-Pentapeptid- und anti-Hexapegtid-Antikörper monoklonale oder polyklonale Antikörper sind.

6. Kit nach einem der Ansprüche 1 bis 5, dadurch gekennzeichnet, daß der anti-Ang II-Antikörper in Lösung vorliegt oder auf einem festen Träger fixiert ist.

7. Kit nach Anspruch 6, dadurch gekennzeichnet, daß der feste Träger ein Röhrchen, eine Mikrotiterplatte oder, gegebenenfalls magnetische, Teilchen sind.

8. Kit nach Anspruch 6, dadurch gekennzeichnet, daß die Antikörper polyklonal sind und jeweils von einem Kaninchen-Antiserum stammen.

9. Antikörper, welcher durch die Immunisierung eines Tieres mit einem Peptid-Derivat der Formel :

$$H-X_1-Tyr-Ile-His-R_1-R_2-Cys-NH_2$$

erhalten wird, worin

- $R_1$ eine direkte Bindung oder eine Kette aus 1 bis 2 natürlichen Aminosäuren bedeutet,
- $R_2$ eine Gruppe der Formel: $-NH-(CH_2)_n-CO-$ darstellt, wobei n zwischen 1 und 9 variiert,
- $X_1$ eine direkte Bindung, Val oder Arg-Val, gemäß der Spezifität des herzustellenden Antiserums, ist,

wobei das Peptid-Derivat auf einem Vektor-Protein fixiert ist, und die Antikörper eine Kreuzreaktion mit Angiotensin II von weniger als 10 %, vorzugsweise weniger als 5 % aufweist.

10. Anti-Ang III-Antikörper nach Anspruch 9, dadurch gekennzeichnet, daß das Peptid-Derivat die Formel:

$$H-Arg-val-Tyr-Ile-His-R_1-R_2-Cys-NH_2 \qquad\qquad (VI)$$

aufweist, worin

- $R_1$ eine direkte Bindung oder eine Kette aus 1 bis 2 natürlichen Aminosäuren bedeutet,
- $R_2$ eine Gruppe der Formel: $-NH-(CH_2)_n-CO-$ darstellt, wobei n zwischen 1 und 9 variiert.

11. Anti-Ang III-Antikörper nach einem der Ansprüche 9 oder 10, dadurch gekennzeichnet, daß das Peptid-Derivat die Formel:

$$H-Arg-Val-Tyr-Ile-His-R'_1-Ahx-Cys-NH_2$$

aufweist, worin

- Ahx den Rest von Amino-6-hexansäure bedeutet,
- und $R'_1$ Pro, oder Pro gebunden an eine andere natürliche Aminosäure, darstellt.

12. Anti-Ang III-Antikörper nach Anspruch 11, dadurch gekennzeichnet, daß er ein Kaninchen-Antiserum ist, und daß er eine Kreuzreaktion mit Ang II von weniger als 1 % aufweist.

13. Anti-Pentapeptid-Antikörper (V) nach Anspruch 9, dadurch gekennzeichnet, daß das Peptid-Derivat die Formel:

$$H-Tyr-Ile-His-R_1-R_2-Cys-NH_2 \qquad\qquad (IX)$$

aufweist, worin

- $R_1$ eine direkte Bindung oder eine Kette aus 1 bis 2 natürlichen Aminosäuren bedeutet,
- $R_2$ eine Gruppe der Formel: $-NH-(CH_2)_n-CO-$ darstellt, wobei n zwischen 1 und 9 variiert.

14. Anti-Pentapeptid-Antikörper (v) nach Anspruch 13, dadurch gekennzeichnet, daß das Peptid-Derivat die Formel:

$$H-Tyr-Ile-His-R'_1-Ahx-Cys-NH_2$$

aufweist, worin

- Ahx den Rest von Amino-6-hexansäure bedeutet,
- und $R'_1$ Pro, oder Pro gebunden an eine andere natürliche Aminosäure, darstellt.

15. Anti-Hexapeptid-Antikörper (IV) nach Anspruch 9, dadurch gekennzeichnet, daß das Peptid-Derivat die Formel:

$$H-Val-Tyr-Ile-His-R_1-R_2-Cys-NH_2 \qquad\qquad (VIII)$$

aufweist, worin

- $R_1$ eine direkte Bindung oder eine Kette aus 1 bis 2 natürlichen Aminosäuren bedeutet,
- $R_2$ eine Gruppe der Formel: $-NH-(CH_2)_n-CO-$ darstellt, wobei n zwischen 1 und 9 variiert.

16. Anti-Hexapeptid-Antikörper (IV) nach Anspruch 15, dadurch gekennzeichnet, daß das Peptid-Derivat die Formel:

$$H-Val-Tyr-Ile-His-R'_1-Ahx-Cys-NH_2$$

aufweist, worin

- Ahx den Rest von Amino-6-hexansäure bedeutet,

- und $R'_1$ Pro, oder Pro gebunden an eine andere natürliche Aminosäure, darstellt.

17. Anti-Ang I-Antikörper, dadurch gekennzeichnet, daß er durch die Immunisierung eines Tieres mit einem Peptid-Derivat der Formel:

$$H\text{-}Cys\text{-}R_2\text{-}R_3\text{-}Arg\text{-}val\text{-}Tyr\text{-}Ile\text{-}His\text{-}Pro\text{-}Phe\text{-}His\text{-}Leu\text{-}OH \hspace{3em} (VII)$$

erhalten wird, worin

- $R_3$ eine direkte Bindung oder eine Kette aus 1 bis 2 natürlichen Aminosäuren bedeutet,
- $R_2$ eine Gruppe der Formel: $-NH\text{-}(CH_2)_n\text{-}CO-$ darstellt, wobei n zwischen 1 und 9 variiert.

18. Antikörper nach Anspruch 17, dadurch gekennzeichnet, daß das Peptid-Derivat die Formel:

$$H\text{-}Cys\text{-}Ahx\text{-}R'_3\text{-}Arg\text{-}Val\text{-}Tyr\text{-}Ile\text{-}His\text{-}Pro\text{-}Phe\text{-}His\text{-}Leu\text{-}OH$$

aufweist, worin

- Ahx den Rest von Amino-6-hexansäure bedeutet,
- $R'_3$ Asp, oder Asp gebunden an eine andere natürliche Aminosäure, darstellt.

19. Verfahren zur Bestimmung von Angiotensin II unter Verwendung des Bestimmungs-Kits nach Anspruch 1, dadurch gekennzeichnet, daß es die folgenden Schritte umfaßt, gemäß denen:

a) ein Reaktionsmedium hergestellt wird, das umfaßt:

- einen Extrakt der zu bestimmenden biologischen Flüssigkeit,
- einen anti-Ang II-Antikörper,
- markiertes Ang II,
- einen anti-Ang III-Antikörper,
- und gegebenenfalls einen anti-Ang I-Antikörper und/oder einen anti-Pentapeptid-Antikörper und/oder einen anti-Hexapeptid-Antikörper;

b) gleichzeitig Reaktionsmedien hergestellt werden, die für eine Standardisierung verwendet werden, und die jeweils umfassen:

- einen Ang II-Standard,
- einen anti-Ang II-Antikörper,
- markiertes Ang II,
- einen anti-Ang III-Antikörper,
- und gegebenenfalls einen anti-Ang I-Antikörper und/oder einen anti-Pentapeptid-Antikörper und/oder einen anti-Hexapeptid-Antikörper;

c) bei einer Temperatur zwischen 4°C und Umgebungstemperatur während 12 bis 72 Stunden inkubieren gelassen wird;
d) an den anti-Ang II-Antikörper gebundenes Ang II von freiem Ang II getrennt wird;
e) schließlich die Ablesung der Ergebnisse gemäß einer geeigneten Methode durchgeführt wird.

20. Verfahren nach Anspruch 19, dadurch gekennzeichnet, daß die Schritte a) und b) durchgeführt werden, indem gemischt werden:

- 25 bis 500 µl Extrakt einer zu bestimmenden biologischen Flüssigkeit, oder Ang II-Standard, mit einem Medium, das enthält:
- 25 bis 500 µl Lösung von anti-Ang II-Antikörper,
- 25 bis 500 µl Lösung von anti-Ang III-Antikörper,
- gegebenenfalls 25 bis 500 µl Lösung von anti-Ang I-Antikörper und/oder 25 bis 500 µl Lösung von anti-Pentapeptid-Antikörper und/oder 25 bis 500 µl Lösung von anti-Hexapeptid-Antikörper, und
- 25 bis 500 µl Lösung von markiertem Ang II.

21. Verfahren nach Anspruch 20, dadurch gekennzeichnet, daß die Schritte a) und b) durchgeführt werden, indem gemischt werden:

- 25 bis 500 µl Extrakt einer zu bestimmenden biologischen Flüssigkeit, oder Ang II-Standard, mit einem Medium, das enthält:
- 25 bis 500 µl Lösung von anti-Ang II-Antikörper,
- 25 bis 500 µl Lösung von anti-Ang III-Antikörper, und
- 25 bis 500 µl Lösung von markiertem Ang II.

22. Verfahren nach Anspruch 19, dadurch gekennzeichnet, daß die Schritte a) und b) durchgeführt werden, indem mit anti-Ang II-Antikörper versehene Röhrchen verwendet werden, in die gemischt werden:

- 25 bis 500 µl Extrakt einer zu bestimmenden biologischen Flüssigkeit, oder Ang II-Standard, mit einem Medium, das enthält:
- 25 bis 500 µl Lösung von anti-Ang III-Antikörper,
- 25 bis 500 µl Lösung von markiertem Ang II,

und Schritt d) durchgeführt wird, indem die Inkubationslösung vom Reaktionsmedium abgesaugt wird, anschließend gewaschen und erneut abgesaugt wird, um Ang II, das nicht durch den auf dem festen Träger fixierten Antikörper komplexiert ist, zu eliminieren.

23. Verfahren nach Anspruch 22, dadurch gekennzeichnet, daß der anti-Ang II-Antikörper auf, gegebenenfalls magnetischen, Teilchen fixiert ist.

24. Verfahren nach Anspruch 23, dadurch gekennzeichnet, daß es zur Bestimmung eines Plasma-Extrakts verwendet wird.

25. Peptid-Derivat der Formel:

$$\text{H-}X_1\text{-Tyr-Ile-His-}R_1\text{-}R_2\text{ -Cys-}NH_2$$

worin

- $R_1$ eine direkte Bindung oder eine Kette aus 1 bis 2 natürlichen Aminosäuren bedeutet, und vorzugsweise $R_1$ Pro, oder Pro gebunden an eine andere natürliche Aminosäure, darstellt,
- $R_2$ eine Gruppe der Formel: $-NH-(CH_2)_n\text{-cO-}$ bedeutet, wobei n zwischen 1 und 9 variiert, und n vorzugsweise 5 ist,
- $X_1$ eine direkte Bindung, einen Valinrest oder einen Arg-Val-Dipeptidrest darstellt.

26. Peptid-Derivat der Formel:

$$\text{H-Cys-}R_2\text{-}R_3\text{-Arg-Val-Tyr-Ile-His-Pro-Phe-His-Leu-OH} \qquad \text{(VII)},$$

worin

- $R_3$ eine direkte Bindung oder eine Kette aus 1 bis 2 natürlichen Aminosäuren bedeutet, und $R_3$ vorzugsweise Asp, oder Asp gebunden an eine andere natürliche Aminosäure, darstellt,
- $R_2$ eine Gruppe der Formel: $-NH-(CH_2)_n\text{-CO-}$ bedeutet, wobei n zwischen 1 und 9 variiert, und n vorzugsweise 5 ist.

## Claims

1. A kit for the specific assay of Angiotensin II, comprising:

- an anti-Ang II antibody,
- a solution of labelled Ang II,
- solutions containing Ang II standards at known and increasing concentrations,
- the requisite washing solution(s),
- a solution of anti-Ang III antibody, the said antibody exhibiting a cross-reaction with Ang II of less than 10% and preferably less than 5%,
- and, optionally, a solution of anti-Ang I antibody and/or a solution of anti-pentapeptide antibody and/or a solution of anti-hexapeptide antibody, the said antibodies exhibiting a cross-reaction with Ang II of less than 10% and preferably less than 5%.

**2.** Kit according to claim 1, characterized in that the Ang II is labelled with an enzyme.

**3.** Kit according to claim 2, characterized in that it comprises, in addition, a solution containing the substrate for the enzyme labelling the Ang II and, optionally, one or more reagents for visualising the activity of the enzyme and a solution intended for stopping the enzymatic reaction.

**4.** Kit according to claim 1, characterized in that the Ang II is labelled either with a fluorescent compound, or with a luminescent compound, or with a radioelement.

**5.** Kit according to any one of claims 1 to 4, in which the anti-Ang II antibody, the anti-Ang III and anti-Ang I, anti-pentapeptide and anti-hexapeptide antibodies are monoclonal or polyclonal antibodies.

**6.** Kit according to any one of claims 1 to 5, characterized in that the anti-Ang II antibody is in solution or is bound to a solid support.

**7.** Kit according to claim 6, characterized in that the solid support is a tube, a microtitration plate or particles, magnetic or otherwise.

**8.** Kit according to claim 6, characterized in that the antibodies are polyclonal and each originate from a rabbit antiserum.

**9.** Antibody obtained by immunisation of an animal with a peptide derivative of formula:

$$H\text{-}X_1\text{-}Tyr\text{-}Ile\text{-}His\text{-}R_1\text{-}R_2\text{-}Cys\text{-}NH_2$$

in which

- $R_1$ denotes a direct bond or a chain-link comprising 1 or 2 natural amino acids,
- $R_2$ represents a group of formula: $-NH-(CH_2)_n-CO-$ in which n varies between 1 and 9,
- $X_1$ represents a direct bond, Val or Arg-Val, depending on the specificity of the antiserum to be prepared,

the said peptide derivative being bound to a carrier protein, said antibody exhibiting a cross-reaction with angiotensin II of less than 10% and preferably less than 5%.

**10.** Anti-Ang III antibody according to claim 9, characterized in that the peptide derivative is of formula:

$$H\text{-}Arg\text{-}Val\text{-}Tyr\text{-}Ile\text{-}His\text{-}R_1\text{-}R_2\text{-}Cys\text{-}NH_2 \qquad\qquad (VI)$$

in which

- $R_1$ denotes a direct bond or a chain-link comprising 1 or 2 natural amino acids,
- $R_2$ represents a group of formula: $-NH-(CH_2)_n-CO-$ in which n varies between 1 and 9.

**11.** Anti-Ang III antibody according to one of claims 9 or 10, characterized in that the peptide derivative has the formula:

$$H\text{-}Arg\text{-}Val\text{-}Tyr\text{-}Ile\text{-}His\text{-}R'_1\text{-}Ahx\text{-}Cys\text{-}NH_2$$

in which

- Ahx denotes the 6-aminohexanoic acid residue,
- and $R'_1$ represents Pro or Pro bound to another natural amino acid.

**12.** Anti-Ang III antibody according to claim 11, characterized in that it is a rabbit antiserum and in that it exhibits a cross-reaction with Ang II of less than 1%.

**13.** Anti-pentapeptide (V) antibody according to claim 9, characterized in that the peptide derivative is of formula:

$$H\text{-}Tyr\text{-}Ile\text{-}His\text{-}R_1\text{-}R_2\text{-}Cys\text{-}NH_2 \qquad\qquad (IX)$$

in which

- $R_1$ denotes a direct bond or a chain-link comprising 1 or 2 natural amino acids,
- $R_2$ represents a group of formula: $-NH-(CH_2)_n-CO-$ in which n varies between 1 and 9.

14. Anti-pentapeptide (V) antibody according to claim 13, characterized in that the peptide derivative has the formula:

$$\text{H-Tyr-Ile-His-R'}_1\text{-Ahx-Cys-NH}_2$$

in which

- Ahx denotes the 6-aminohexanoic acid residue,
- and $R'_1$ represents Pro or Pro bound to another natural amino acid.

15. Anti-hexapeptide (IV) antibody according to claim 9, characterized in that the peptide derivative is of formula:

$$\text{H-Val-Tyr-Ile-His-R}_1\text{-R}_2\text{-Cys-NH}_2 \qquad \text{(VII)}$$

in which

- $R_1$ denotes a direct bond or a chain-link comprising 1 or 2 natural amino acids,
- $R_2$ represents a group of formula: $-NH-(CH_2)_n-CO-$ in which n varies between 1 and 9.

16. Anti-hexapeptide (IV) antibody according to claim 15, characterized in that the peptide derivative has the formula:

$$\text{H-Val-Tyr-Ile-His-R'}_1\text{-Ahx-Cys-NH}_2$$

in which

- Ahx denotes the 6-aminohexanoic acid residue,
- and $R'_1$ represents Pro or Pro bound to another natural amino acid.

17. Anti-Ang I antibody, characterized in that it is obtained by immunisation of an animal with a peptide derivative of formula:

$$\text{H-Cys-R}_2\text{-R}_3\text{-Arg-Val-Tyr-Ile-His-Pro-Phe-His-Leu-OH} \qquad \text{(VII)}$$

in which

- $R_3$ denotes a direct bond or a chain-link comprising 1 or 2 natural amino acids,
- $R_2$ represents a group of formula: $-NH-(CH_2)_n-CO-$ in which n varies between 1 and 9.

18. Antibody according to claim 17, characterized in that the peptide derivative has the formula:

$$\text{H-Cys-Ahx-R'}_3\text{-Arg-Val-Tyr-Ile-His-Pro-Phe-His-Leu-OH}$$

in which

- Ahx denotes the 6-aminohexanoic acid residue,
- and $R'_3$ represents Asp or Asp bound to another natural amino acid.

19. Method for the assay of angiotensin II in which the assay kit according to claim 1 is used, characterized in that it comprises the following steps:

a) the reaction medium comprising the following is prepared:

- the extract of biological fluid to be assayed,
- an anti-Ang II antibody,
- labelled Ang II,
- an anti-Ang III antibody,
- and, optionally, an anti-Ang I antibody and/or an anti-pentapeptide antibody and/or an anti-hexapeptide antibody;

b) the reaction media useful for the calibration, each comprising the following, are prepared simultaneously:

- an Ang II standard,
- an anti-Ang II antibody,
- labelled Ang II,
- an anti-Ang III antibody,
- and, optionally, an anti-Ang I antibody and/or an anti-pentapeptide antibody and/or an anti-hexapeptide antibody;

c) the media are left to incubate at a temperature between 4°C and room temperature for 12 to 72 hours;

d) the Ang II bound to the anti-Ang II antibody is separated from the free Ang II;

e) lastly, the reading of the results is performed according to a suitable method.

**20.** Method according to claim 19, characterized in that steps a) and b) are carried out by mixing:

- 25 to 500 μl of extract of biological fluid to be assayed, or of Ang II standard, with a medium containing:
- 25 to 500 μl of solution of anti-Ang II antibody,
- 25 to 500 μl of solution of anti-Ang III antibody,
- optionally, 25 to 500 μl of solution of anti-Ang I antibody and/or 25 to 500 μl of solution of anti-pentapeptide antibody and/or 25 to 500 μl of solution of anti-hexapeptide antibody, and
- 25 to 500 μl of solution of labelled Ang II.

**21.** Method according to claim 20, characterized in that steps a) and b) are carried out by mixing:

- 25 to 500 μl of extract of biological fluid to be assayed, or of Ang II standard, with a medium containing:
- 25 to 500 μl of solution of anti-Ang II antibody,
- 25 to 500 μl of solution of anti-Ang III antibody, and
- 25 to 500 μl of solution of labelled Ang II.

**22.** Method according to claim 19, characterized in that steps a) and b) are carried out using tubes coated with anti-Ang II antibody, in which:

- 25 to 500 μl of extract of biological fluid to be assayed, or of Ang II standard are mixed with a medium containing:
- 25 to 500 μl of solution of anti-Ang III antibody,
- 25 to 500 μl of solution of labelled Ang II,

and step d) is carried out by aspiration of the incubation solution from the reaction medium, followed by washing and further aspiration in order to remove Ang II which is not complexed by the antibody bound to the solid support.

**23.** Method according to claim 22, characterized in that the anti-Ang II antibody is bound to particles, magnetic or otherwise.

**24.** Method according to claim 23, characterized in that it is used for the assay of a plasma extract.

**25.** Peptide derivative of formula:

$$\text{H-X}_1\text{-Tyr-Ile-His-R}_1\text{-R}_2\text{-Cys-NH}_2$$

in which

- $R_1$ denotes a direct bond or a chain-link comprising 1 or 2 natural amino acids, preferably $R_1$ represents Pro or Pro bound to another natural amino acid,
- $R_2$ represents a group of formula: $\text{-NH-(CH}_2)_n\text{-CO-}$ in which n varies between 1 and 9, preferably n is 5,
- $X_1$ represents a direct bond, the valine residue or the residue of the dipeptide Arg-Val.

**26.** Peptide derivative of formula:

$$\text{H-Cys-R}_2\text{-R}_3\text{-Arg-Val-Tyr-Ile-His-Pro-Phe-His-Leu-OH} \qquad \text{(VII)}$$

in which

- $R_3$ denotes a direct bond or a chain-link comprising 1 or 2 natural amino acids, preferably $R_3$ represents Asp or Asp bound to another natural amino acid,
- $R_2$ represents a group of formula: $\text{-NH-(CH}_2)_n\text{-CO-}$ in which n varies between 1 and 9, preferably n is 5.